# EUROPEAN PATENT APPLICATION

(11) **EP 1 674 116 A2**
(43) Date of publication of application: **28.06.2006**
(21) Application number: 05257908.3
(22) Date of filing: 21.12.2005
(51) Int. Cl.: A61L 27/24, A61L 27/36

(54) **Self-assembling protein matrix prepared from natural extracellular matrices**

(30) Priority: 22.12.2004 US 22517
(71) Applicant: DePuy Products, Inc., Warsaw, IN 46581 (US)
(72) Inventor: Pedrozo, Hugo A., Silver Lake IN 46982 (US); Shuster, Mark, Toledo, OH 43614 (US)
(74) Representative: Belcher, Simon James

(57) **Abstract**

A method for preparing a biomaterial involving extracting Collagen Type I (Col I) protein from small intestine submucosa (SIS) under conditions that preserve the native helical configuration, involves extracting collagen Type I protein from submucosal tissue using an acidic reagent selected from the group consisting of acetic acid, citric acid, or formic acid, in the absence of detergents or enzymes, to produce a solution of collagen fibres wherein greater than 75% of the fibres have an α-helical tertiary structure. Self assembly of the collagen fibres to form the biomaterial is then induced, for example by adding a salt solution to the extract.

## Description

The present invention relates to a protein polymer and a method for preparing the polymer from a self-assembling protein. The protein polymers prepared in accordance with the present invention are useful in biomedical applications.

Successful cell invasion, attachment, and proliferation are all prerequisites for successful remodeling of any resorbable implant or acceptance of any non-resorbable implant surface. However, the process of cell differentiation, which follows cell proliferation, in essence, determines the long-term success of the implant. For example, successful invasion and attachment of periosteal cells into a bone implant followed by fast proliferation does not necessarily lead to their differentiation into osteoblastic (bone forming) cells. Since cell differentiation is what affords functionality to any tissue, i.e. the ability of tissue to perform a task is directly related to the cell types that form it and their ability to work in concert, it is important to gain improvement on the cell differentiation processes.

It has been known that Collagen foams, gels and scaffolds may be used as scaffolds for cell attachment and proliferation and, in some instances, for drug delivery. Collagen Type I (Col I) is the main structural protein of higher organisms, and Col I scaffolds can be fabricated using such Col I molecules, which are capable of self assembling. Col I constitutes the basic scaffold upon which cells deposit other extra-cellular matrix components specific to their phenotype and function, and Col I scaffolds may affect cell differentiation. Col I is an intra-cellularly assembled heterotrimeric complex composed of two a and one α2 chains each with an alpha helical secondary structure that allows them to wind around each other to make long extra-cellular filaments. These filaments (each being a Col I molecule) assemble with each other forming long fibers that can be greater than 1.0 µm in length. Because the molecular forces that orchestrate this process are weak and because the α-helical (strand-like) nature of the α1 and α2 chains is not canonical, their assembly can easily turn into an unstructured aggregation of misfolded proteins.

In the literature, there are known methods for isolating Col I from a variety of tissues, e.g. placenta and tails, and using the isolated material to reconstitute collagenous matrices. These known methods rely on the protein's intrinsic ability to retain its secondary structure during protein isolation and assume that, for instance, the alpha helix will retain its helical structure throughout. The end result, even with a homogenous biochemical composition, can be a heterogeneous secondary structure. Controlling the assembly of the constituting monomers into tertiary or quaternary multimeric arrangements is very hard to achieve under such conditions.

In one aspect, the invention provides a method for isolating a substantially pure composition of collagen type I fibres and the use of such a composition to prepare a biomaterial. More particularly, in accordance with one embodiment a method for preparing a biomaterial comprises extracting collagen type I (Col I) protein from a naturally occurring extracellular matrix under conditions that preserve the native helical configuration. The extract produced by the method of the present invention is composed almost entirely of collagen type I. In accordance with one embodiment the naturally occurring extracellular matrix comprises small intestine submucosa, however it is contemplated that other sources of naturally occurring extracellular matrices, such as placenta or tails of a swine or other animals, may also be used as the starting materials.

Preferably, the small intestinal submucosa from a swine is homogenized in an acidic reagent, having a pH of about 2 to about 3.5. The homogenized mixture, in accordance with one embodiment, is incubated in ice while the protein is extracted into the solution. More particularly, in accordance with one embodiment a method for preparing a collagen Type I extract from a naturally occurring extracellular matrix comprises extracting submucosal tissue using an acidic reagent selected from the group consisting of acetic acid, citric acid, or formic acid, in the absence of detergents or enzymes, to produce a solution of collagen fibres wherein greater than 75% of the fibres have an α-helical tertiary structure. The protein extract is then separated from the debris.

Preferably, a method of forming a collagen type I matrix from the Col I extracts of the present invention is provided, comprising the steps of subjecting the Col I extract to a temperature of above 20°C, but less than 40°C to induce protein assembly to form fibrillar arrangements of the protein into polymeric structures. In addition, a salt solution may be added to the protein extract to accelerate the self-assembling process. Suitable salt solutions include ammonium sulphate solution, sodium phosphate solution, phosphate buffered saline (PBS) solution or sodium chloride solution. A solution of ammonium sulphate at a concentration of about 0.03 M to 0.09 M is particularly suitable for inducing the polymer formation. In an alternative embodiment, a Col I solution can be induced to form polymeric structures by freezing the extract at -70°C for 1 to 12 hours followed by drying the composition in a vacuum.

Protein assembling may be induced in the presence of a bioactive agent or cells, wherein the bioactive agent or cells become attached to or embedded in the protein polymer during the polymer formation. Alternatively, the bioactive agent or cells may be added after the polymer is already formed. In a further embodiment, an extract containing Col I fibers may be applied to a surface and induced to form a polymer directly on the surface by adding a salt solution during or after application of the Col I fibers on the surface. The application of the Col I fiber extract and the salt solution to the surface may be performed using a pressurized delivery device under a pressure of about 0.034 MPa to about 0.17 MPa (about 5 psi to about 25 psi). In one embodiment, the Col I fibre extract may be premixed with the salt solution prior to the application to the surface. In addition, a bioactive agent and/or cells may be added to the extract and the salt solution premix before the final mixture is applied to the surface. The surface may be a wound or a surface of a biomedical device.

One embodiment of the present invention is directed to a kit for applying a collagen matrix on a surface *in situ.* In one embodiment the kit comprises a collagen type I solution and a salt solution comprising a salt selected from the group consisting of ammonium sulphate solution, sodium phosphate solution, phosphate buffered saline (PBS) solution and NaCl solution. More particularly in one embodiment the collagen type I solution comprises collagen that is isolated from a natural extracellular matrix, by extracting the natural matrix with an acidic reagent, selected from the group consisting of acetic acid, citric acid, or formic acid, at a pH of about 2 to about 3.5. Typically this extraction is performed in the absence of detergents and at temperature of less than 20°C. The kit may contain additional components such as a bioactive agent, cells and/or a device for the mixing and dispensing of the collagen and salt solutions of the kit.

One embodiment of the present invention encompasses the Col I extract and the collagen type I matrix prepared in accordance with the methods described herein, as well as any biomedical devices coated or otherwise combined with such materials.

In describing and claiming the invention, the following terminology will be used in accordance with the definitions set forth below.

A used herein the term "bioactive agents" include one or more of the following:
chemotactic agents; therapeutic agents (e.g. antibiotics, steroidal and non-steroidal analgesics and anti-inflammatories, anti-rejection agents such as immunosuppressants and anti-cancer drugs); various proteins (e.g. short chain peptides, bone morphogenic proteins, glycoprotein and lipoprotein); cell attachment mediators; biologically active ligands; integrin binding sequence; ligands; various growth and/or differentiation agents (e.g. epidermal growth factor, IGF-I, IGF-II, TGF-β I-III, growth and differentiation factors, vascular endothelial growth factors, fibroblast growth factors, platelet derived growth factors, insulin derived growth factor and transforming growth factors, parathyroid hormone, parathyroid hormone related peptide, bFGF; TGFß superfamily factors; BMP-2; BMP-4; BMP-6; BMP-12; sonic hedgehog; GDF5; GDF6; GDF8; PDGF); small molecules that affect the upregulation of specific growth factors; tenascin-C; hyaluronic acid; chondroitin sulphate; fibronectin; decorin; thromboelastin; thrombin-derived peptides; heparin-binding domains; heparin; heparan sulphate; DNA fragments and DNA plasmids, steroidal and non-steroidal hormones such as vitamin D, ecosanoinds, immuno modulators such as IL10, IL4 and IL12, and synthetic anti-inflammatory and immunomodulating agents such as analgesics, **p38** regulators, and other regulators of TNFα up and downstream signalling.

As used herein the term "collagen-based matrix" refers to extracellular matrices that comprise collagen fibres and include both naturally occurring extracellular matrices as well as reconstituted collagen matrices.

As used herein the term "naturally occurring extracellular matrix" comprises any noncellular material naturally secreted by cells (such as intestinal submucosa) isolated in their native configuration with or without naturally associated cells.

As used herein the term "submucosal matrices" refers to natural extracellular matrices, known to be effective for tissue remodelling, that have been isolated in their native configuration, including submucosa derived from intestinal tissue (autograft, allograft, and xenograft), stomach tissue (autograft, allograft, and xenograft), bladder tissue (autograft, allograft, and xenograft), alimentary tissue (autograft, allograft, and xenograft), respiratory tissue (autograft, allograft, and xenograft) and genital tissue (autograft, allograft, and xenograft), and derivatives of liver tissue (autograft, allograft, and xenograft), including for example liver basement membrane.

As used herein the term "exogenous" or "exogenously added" designates the addition of a new component to a composition, or the supplementation of an existing component already present in the composition, using material from a source external to the composition.

As used herein the term "collagen type I matrix" refers to a polymer complex that comprises collagen fibres, wherein greater than 75% of the collagen fibres have an α-helical tertiary structure.

As used herein the term "cells", refers to eukaryotic cells and absent any further elaboration/characterization, includes one or more of the following: chondrocytes; fibrochondrocytes; osteocytes; osteoblasts; osteoclasts; synoviocytes; bone marrow cells; mesenchymal cells; stromal cells; stem cells; embryonic stem cells; precursor cells derived from adipose tissue; peripheral blood progenitor cells; stem cells isolated from adult tissue; genetically transformed cells; a combination of chondrocytes and other cells; a combination of osteocytes and other cells; a combination of synoviocytes and other cells; a combination of bone marrow cells and other cells; a combination of mesenchymal cells and other cells; a combination of stromal cells and other cells; a combination of stem cells and other cells; a combination of embryonic stem cells and other cells; a combination of precursor cells isolated from adult tissue and other cells; a combination of peripheral blood progenitor cells and other cells; a combination of stem cells isolated from adult tissue and other cells; and a combination of genetically transformed cells and other cells. If other cells are found to have therapeutic value in the orthopaedic field, it is anticipated that at least some of these cells will have use in the present invention, and such cells should be included within the meaning of "cell" and "cells" unless expressly limited otherwise. Illustratively, in one example of embodiments that are to be seeded with living cells such as chondrocytes, a sterilized implant may be subsequently seeded with living cells and packaged in an appropriate medium for the cell type used. For example, a cell culture medium comprising Dulbecco's Modified Eagles Medium (DMEM) can be used with standard additives such as non-essential amino acids, glucose, ascorbic acid, sodium pyruvate, fungicides, antibiotics, etc., in concentrations deemed appropriate for cell type, shipping conditions, etc.

The present method provides a stable, raw biomaterial that can be used to engineer scaffolds and surfaces with the "right" architecture for specific loads or strains to promote accelerated cell proliferation and differentiation. The biomaterial can be manipulated using inexpensive, non-toxic, and environmentally sound means. In accordance with one embodiment the present invention provides a method of isolating collagen type I proteins from naturally occurring materials for use in the *in situ* delivery of a self-assembling collagen type I matrix.

In accordance with one embodiment the self-assembling protein matrix of the present invention is prepared using a purified collagen type I composition that is prepared from natural materials that have a high collagen content, including naturally occurring extracellular matrices. In one embodiment the method for preparing a composition comprising a high content of collagen type I fibres comprises the steps of extracting collagen type I protein from a naturally occurring extracellular matrix, including for example submucosal tissue , through the use of an acidic reagent selected from the group consisting of acetic acid, citric acid, or formic acid. The extraction of the collagen type I protein from the natural material is conducted in the absence of detergents or enzymes. In accordance with one embodiment the starting material is extracted using acetic acid at a pH of about 2 to about 3.5, and the temperature is kept below 20°C during the extraction procedure.

In accordance with one embodiment the starting material is comminuted by tearing, cutting, grinding, shearing and the like to enhance the extraction of the collagen type I protein from the starting material. In one embodiment, the starting material is ground in a frozen or freeze-dried state, and in another embodiment the starting material is homogenized in a high speed (high shear) blender, and dewatering, if necessary, by centrifuging and decanting excess water. The starting material can be comminuted in the presence of the acidic reagent, or the material can be first comminuted and then contacted with the acidic reagent.

In accordance with one embodiment the starting material is homogenized in the presence of about 0.1 to about 1.0 M acetic acid, and in one embodiment 0.5 M acetic acid, while the temperature of the homogenate is maintained below 20°C, and typically the homogenate temperature is kept near 4°C, through out the extraction process. In accordance with one embodiment the starting tissue is subjected to repeated cycles of homogenization in the presence of the acidic reagent, wherein the homogenate is placed on ice in between the homogenization steps.

The collagen type I comprising fraction is recovered by removing the insoluble fractions, typically by centrifugation and recovery of the supernatant. In accordance with one embodiment the extracted starting material is subjected to centrifugation at about 3000 x g and the supernatant is recovered. However, other separation techniques such as filtration, and other techniques known to the skill practitioner, can be used in accordance with the present invention to recover the soluble fraction.

The extraction procedures of the present invention produce a composition that is substantially pure collagen type I in its native α-helical structure, with a low amount of other proteinaceous constituents having β-sheet and random coil secondary structures. In accordance with one embodiment the resulting collagen extract comprises 75%, 85%, 90%, 95%, 98% or higher collagen type I in its native α-helical structure.

In accordance with one embodiment the starting material used to isolate the high content collagen type I solution is a tissue that initially contains high collagen type I content. For example, placenta and mammalian tails are known to contain large amounts of collagen type I. In one embodiment the starting material comprises a naturally occurring extracellular matrix. Extracellular matrices isolated from various tissues are known to be effective for tissue remodelling, and include, but are not limited to, extracellular matrices isolated from mammalian intestine, stomach, bladder, alimentary, respiratory, and genital submucosa, for example as disclosed in US-4902508, US-6171344, US-6099567 and US-5554389. These tissues are referred to generally as "submucosal matrices" and comprise highly conserved collagens, glycoproteins, proteoglycans, and glycosamino-glycans. Additionally, other known extracellular matrices, for example lamina propria and stratum compactum, may also be used in accordance with the present invention.

In one embodiment the staring material comprises intestine submucosa, and in a further embodiment the layer comprises small intestinal submucosa of a warm blooded vertebrate. In one embodiment, the material comprises the tunica submucosa along with the lamina muscularis mucosa and the stratum compactum of a segment of intestine, said layers being delaminated from the tunica muscularis and the luminal portion of the tunica mucosa of said segment. Such a material is referred to herein as intestinal submucosa (SIS). In accordance with one embodiment of the present invention the intestinal submucosa comprises the tunica submucosa along with basilar portions of the tunica mucosa of a segment of intestinal tissue of a warm-blooded vertebrate. While porcine SIS is widely used, it will be appreciated that intestinal submucosa may be obtained from other animal sources, including cattle, sheep, and other warm-blooded mammals.

The preparation of SIS from a segment of small intestine is disclosed in US-4902508. A segment of intestine is first subjected to abrasion using a longitudinal wiping motion to remove both the outer layers (particularly the tunica serosa and the tunica muscularis) and the inner layers (the luminal portions of the tunica mucosa). Typically the SIS is rinsed with saline and optionally stored in a hydrated or dehydrated state until use. Details of the characteristics and properties of intestinal submucosa (SIS) are disclosed in US-4352463, US-4902508, US-4956178, US-5281422, US-5372821, US-5445833, US-5516533, US-5573784, US-5641518, US-5645860, US-5668288, US-5695998, US-5711969, US-5730933, US-5733868, US-5753267, US-5755791, US-5762966, US-5788625, US-5866414, US-5885619, US-5922028, US-6056777 and WO-97/37613. SIS, in various forms, is commercially available from Cook Biotech Incorporated (Bloomington, Ind.).

In one embodiment an intestinal submucosa matrix is used as the starting material, and the material is comminuted by tearing, cutting, grinding, shearing and the like in the presence of an acidic reagent selected from the group consisting of acetic acid, citric acid, and formic acid. In one embodiment the acidic reagent is acetic acid. In one embodiment, the intestinal submucosa is ground in a frozen or freeze-dried state to prepare a comminuted form of SIS. Alternatively, comminuted SIS can also be obtained by subjecting a suspension of pieces of the submucosa to treatment in a high speed (high shear) blender, and dewatering, if necessary, by centrifuging and decanting excess water.

The material extracted from SIS (named SISH) is mostly composed of Collagen Type I. This material has been found to be stable in solution at 4°C for weeks.

The collagenous extracts of the present invention allow collagen type I matrices to be formed, wherein the matrix formed is stable in the presence of acidic solutions. More particularly, the acid stable collagen type I matrices of the present invention can be formed *in situ.* This capability advantageously allows for collagenous matrices to be coated onto surfaces *in situ* wherein the matrix conforms to the shape of the underlying material, while providing a structurally stable matrix. Furthermore, the matrix can be prepared to include bioactive agents or cells and thus it can function as a delivery system whose assembly will *occur in situ.* Self-assembly of the collagen Type I matrix is permitted and encouraged by an increase in the entropy of its environment, which in one embodiment is done by raising the temperature to above 20°C, but below 40°C, and in one embodiment raising the temperature to about 34°C to about 38°C. In one embodiment self assembly is induced by raising the temperature of the collagen extract to about 37°C.

In accordance with one embodiment self assembly is further encouraged by the addition of small amounts of salts, such as ammonium sulphate solution, sodium phosphate solution, phosphate buffered saline (PBS) solution and NaCl solution or a combination thereof. The addition of small amounts of salts speeds the formation of the collagenous matrix without significantly impacting the desirable properties of the formed matrix. In accordance with one embodiment the salt is ammonium sulphate or sodium phosphate or a combination of the two. Typically, ammonium sulphate or sodium phosphate is added to the collagen extract in final concentration of about 1mM to about 100mM. In one embodiment the salt used is ammonium sulphate, and in one particular embodiment ammonium sulphate is added to a final concentration of about 0.3 mM to about 90 mM. In one embodiment ammonium sulphate is added to a final concentration of about 3.0 mM to about 30 mM.

In accordance with one embodiment a method of assembling a collagen type-I matrix on a surface *in situ* is provided. The method comprises the steps of applying a composition comprising a collagen type I solution to a surface, wherein the collagen type I solution is prepared by extracting a naturally occurring extracellular matrix with acetic acid at a pH of about 2 to about 3.5. A salt solution is then contacted with the collagen type I solution to induce collagen type I self assembly resulting in a collagen type-I matrix formed on the surface. In one embodiment the collagen composition and the salt solution are combined to form a mixture, and the mixture is applied to the surface.

In accordance with one embodiment various bioactive agents or cells are added to the collagen/salt mixture before the mixture has assembled into the collagen type-I matrix. Alternatively, the bioactive agent or cells can be adhered to the fibres of the collagen type-I matrix after self assembly. In one embodiment the bioactive agent is a growth factor. In this manner the matrix can be used as a delivery vehicle for delivering compounds and/or cells to a specific location. The material can be deposited on a metal or plastic prosthesis or on a biological surface. For example, the material can be applied to a wound, muscle, epithelium, periosteum, bone or cartilage during surgery or onto an implantable device or material.

The bioactive agent can be adsorbed onto a formed collagen matrix or be mixed with the collagen extract prior to self assembly, wherein induction of self assembly entraps the bioactive agent within the matrix. The approach taken depends on the molecule to be used, its hydrophobicity, structure, size, concentration and ability to interact with collagen. In embodiments where the bioactive agent is mixed with the components prior to self assembly, the composition comprising the bioactive agent can be premixed with the salt solution, premixed with the collagen solution in acetic acid, or the three solutions can be simultaneously mixed together.

The properties of the resulting self assembled collagenous matrix can be altered by modifying the concentration of the collagenous solution or the salt concentration. Typically, the concentration of collagen ranges from about 0.05 mg/ml to about 1.5 mg/ml, with higher concentration of collagen giving rise to material having higher tensile strength.

In accordance with one embodiment the collagen solution and the salt solution are mixed in a device that allows the deposition of the mixture while the collagen is undergoing self assembly. In one embodiment the collagen solution and salt solution are mixed in a pressurized device and the self assembling mixture is extruded under pressure. More particularly, in one embodiment a device comprising a multi-chambered assembly for holding the collagen solution and the salt solution and optionally additional solutions is provided, wherein each chamber is provided with a valve that allows fluid communication with a single mixing chamber and the mixing chamber is provided with a nozzle for releasing materials from the mixing chamber. In one embodiment a port is provided in said mixing chamber that communicates with a source of positive pressure (i.e. a compressor or pressurized gas cartridge or tank). Activation of the device results in the solutions being introduced into the mixing chamber and out through the nozzle, leading to the self-assembly of collagen as the liquid material is ejected onto a target surface.

At a minimum, the device is provided with at least two-reservoir chambers, one for the collagen solution and one for the salt solution. A third reservoir chamber is provided for those embodiment where a bioactive agent or cells are added to the mixture prior to formation of the collagen matrix. In accordance with one embodiment the device is used to 'paint' the collagen matrix onto the desired surface, such as bony surface *in situ.* This would require low pressure (0.034 MPa (5 psi)) to gently coax the collagen matrix out a fanned tip and paint the collagen mixture onto the surface. In this embodiment the collagen mixture is placed under a much higher pressure of about 0.10 MPa to about 0.17 MPa (about 15 psi to about 25 psi). The nozzle of the mixing chamber can be modified to have different types of ejection tips to achieve different types of ejection, i.e. a fanned tip, or spray nozzle tip, or specially designed tips to fit specific body cavities.

There are many potential applications of a device and method for delivering a collagen type I matrix to a biological surface. In one embodiment the collagen matrix is used as a delivery vehicle for various bioactive agents, including growth factors or blockers / inhibitors. Alternatively, the collagenous matrix could be used for joint resurfacing. This would be optimal for locations where there are limited bony surfaces to attach a prosthetic device, such as the glenoid cavity of the shoulder. Ideally, both applications could be used in tandem, where a joint could be resurfaced at an accelerated rate through use of a growth factor and/or cells. The scaffold set up by the sprayed collagen would allow cells to attach and proliferate, and thus biologically resurface the joint. In one embodiment cells are added to the collagenous matrix as it is being self assembled and applied in situ.

As a drug delivery system, the collagen type I matrices can be used to design structural systems that can be made to assemble *in situ* with interpenetration into the native tissue and conforming to the geometry of the anatomical site where the drug is to be delivered. Furthermore, as structural scaffolds, the material can be used to engineer macromolecular complexes suitable to support the bulk of the mechanical demands of the environment in which they are implanted.

In accordance with one embodiment a kit is provided for forming collagen Type I matrices on surfaces *in situ.* The kit comprises reagents for preparing collagen type I matrices, and to this end, the reagents can be packaged in a variety of containers, such as vials, tubes, bottles, and the like. In one embodiment the kit includes disposable cartridges that comprise component reagents, wherein the cartridges can be fitted into a device that allows the reagents to be dispensed from the cartridges, mixed and applied as a mixture onto a surface. The kit will also be provided with instructional materials for preparing collagen matrices using the reagents and other components of the kit.

In one embodiment the kit comprises two components: a composition comprising collagen wherein at least 75% of the collagen has an α-helical tertiary structure and a composition comprising a salt solution selected from the group consisting of ammonium sulphate solution, sodium phosphate solution, phosphate buffered saline (PBS) solution and NaCl solution or a combination thereof. In one embodiment the collagen solution comprises collagen that is isolated from a natural extracellular matrix using steps comprising extracting the natural matrix with an acidic reagent, selected from the group consisting of acetic acid, citric acid, or formic acid, at a pH of about 2 to about 3.5 and a temperature of less than about 30°C, and isolating the soluble portion. In one embodiment the salt solution comprising a salt selected from the group consisting of ammonium sulphate solution or sodium phosphate solution. The kit may also be provided with one or more cell types and/or bioactive agents, including for example various growth factors.

In one embodiment the collagen solution comprises a substantially homogeneous composition comprising collagen type I protein in a native helical configuration, wherein said solution is prepared from a natural extracellular matrix by extracting the natural tissue with an acidic solution of about 2 to about 3.5 pH at a temperature of less than 20°C, and in the absence of detergents or enzymes. The collagen in the starting material may be extracted with an acidic reagent, having an acidic pH (pH of about 2 to about 3.5). The acidic reagent may include but is not limited to acids such as acetic acid, citric acid, formic acid or hydrochloric acid. The extraction procedure generally involves macerating the tissue in small pieces prior to homogenizing the tissue in the extracting reagent. The tissue may be incubated in the reagent in ice at 4°C or at room temperature (about 20°C) for a period of several minutes or several hours to enhance the amount of protein extracted into the reagent. At the end of the extraction process, the tissue debris is separated from the extract and discarded. A specific example of the extraction process is described in Example 1.

In accordance with one embodiment the kit comprises a collagen solution and a salt solution, wherein the collagen solution comprises collagen type I isolated from a submucosal matrix by steps comprising homogenizing the submucosal matrix in an acetic acid solution, and isolating the soluble portion and the salt solution is an ammonium sulphate solution. In one embodiment the collagen solution comprises collagen type I isolated from a submucosal matrix, and in one embodiment, SIS.

The kit of the present invention can be further provided with a device that allows for the mixing and dispensing of the collagen and salt solutions of said kit.

Embodiments of the invention will now be described by way of example with reference to the accompanying drawings, in which:
Figs. 1A & 1B are graphs showing high performance liquid chromatography (HPLC) profiles of two different preparations of collagen type I. Fig. 1A represents an HPLC profile of a 50 µl (about 20 µg) H solution; Fig. 1B represents an HPLC profile of a 145 µl (about 20 µg) sample of a commercially available purified collagen type I typically used for tissue culture, sold under the trade mark VITROGEN.
Fig. 2 is a device for applying a protein mixture of the present invention to a target surface; and
Fig. 3 is another device for applying a protein mixture of the present invention to a target surface.

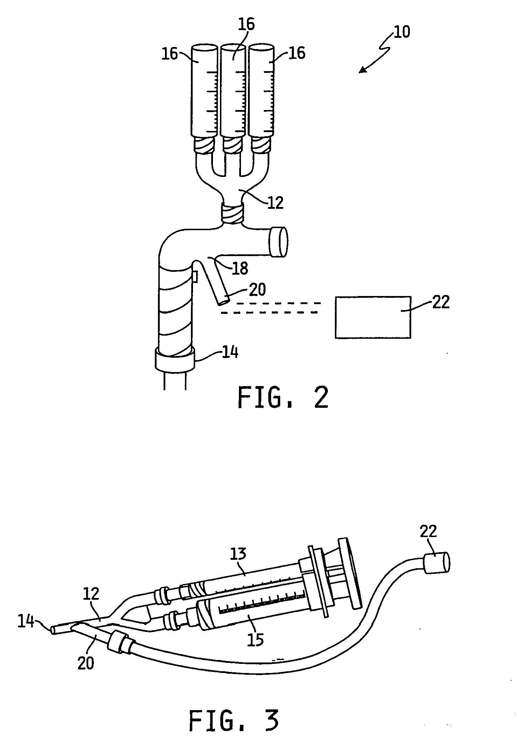

Referring to the drawings, Fig. 2 and Fig. 3 show a device (10) is provided with a mixing chamber (12), a nozzle (14) and a plurality reservoirs (16), wherein each of said reservoirs can be placed in fluid communication with said mixing chamber(12), said mixing chamber being provided with a nozzle (14) that provides a passageway for material to move from the mixing chamber to the exterior, and a port (18) and port extension (20) formed on the mixing chamber that is designed for the attachment of a source of pressurized air (22) to the port extension (20) allowing fluid communication between the source of pressurized air (22) and the mixing chamber (12). In one embodiment the device is further provided with plungers to assist moving the fluid in reservoirs **(16)** into mixing chamber (12) and out nozzle (14). In one embodiment the collagen solution and the salt solution are prepackaged in the reservoirs of the device.

In accordance with the present invention the components of the kit are used to assemble a collagen matrix delivery system *in situ.* The method comprises the steps of providing a composition comprising collagen extracted from a naturally occurring extracellular matrix with a weak acid (such as acetic, citric, or formic acid), mixing the extracted collagen solution with a salt solution at a temperature ranging from 30-40°C and applying the mixture on a solid surface, wherein a type I collagen matrix is formed on the surface. In one embodiment the method further comprises the step of mixing a composition comprising a bioactive agent and or cells with the collagen solution and the salt solution prior to applying the mixture on a solid surface.

### Example 1

### COL I Extraction

The SIS tissue from a swine was washed with peracetic acid diluted with disinfected and reverse osmosis water (RO-water), and about 2 grams (wet weight) of material was cut into small pieces. The SIS pieces were homogenized in 20 ml of 0.5 M acetic acid (ACS reagent grade) for 30 seconds at 9500 RPM and cooled in ice for 60 seconds. Acetic acid ranging anywhere from about 0.1 to about 1 M could also be used to extract collagen from the SIS. The homogenizing and cooling cycle was repeated two to three times. The homogenizer blade was washed, while running, twice with 10 ml of 0.5 M acetic acid and the wash was added to the homogenate (final volume of 40 ml). The homogenate was then centrifuged at 4000 RPM in a Beckman table-top centrifuge (~3000 g) and the supernatant was poured out into a separate 50 ml conical tube and stored at 4°C as the main stock. This protein extract was named SISH solution. The total protein concentration of serial dilutions and of the main stock was determined immediately following sample preparation. About 1 to 1.5 mg.ml⁻¹ (a total of 40 ml) of protein was obtained from the original 2 g of the SIS material.

### Example 2

### Determination of SISH Content

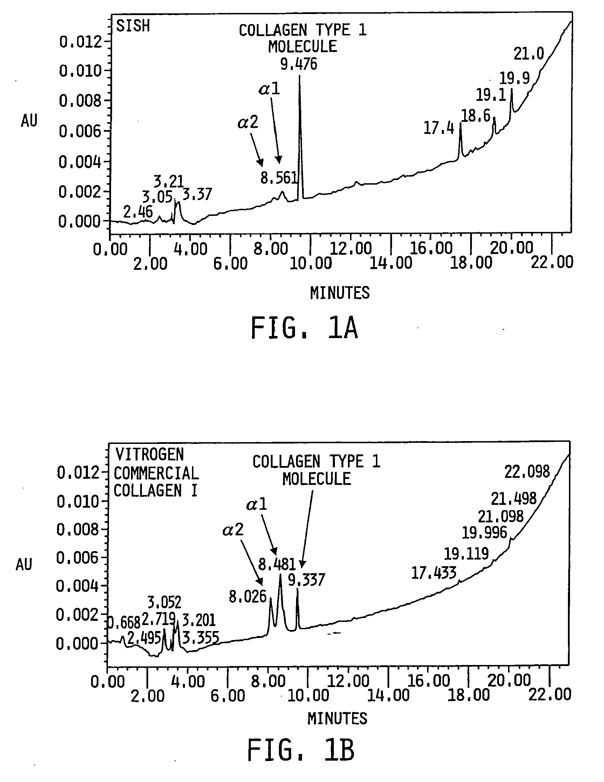

The content of the SISH solution was determined using various analysis techniques such as reduced sodium dodecyl sulphate polyacrylamide gel electrophoresis (SDS-PAGE), non reducing SDS-PAGE and high performance liquid chromatography (HPLC) analysis using a gradient flow of acetonitrile (ACN) in water with 0.1 % trifluoroacetic acid (TFA). It was determined that the SISH solution contains other proteins as well as Col I. In Figs. 1A & 1B, the HPLC profile of the SISH solution (Fig. 1A) is compared with the HPLC profile of a solution of collagen solution (using the material sold by Collagen Corporation of Palo Alto, California under the trade mark Vitrogen) (Fig. 1B). It is notable that the profile of the SISH solution in Fig. 1A shows a tall peak representing a substantial amount of Col I molecules. Each of Col I molecule is a heterotimeric complex composed of two α1 and one α2 chains with an alpha helical secondary structure (the native configuration). The profile also shows two low peaks representing small amounts of the α1 and α2 chains. In contrast, the HPLC profile of the Vitrogen material shown in Fig. 1B shows three peaks of substantially the same height. These peaks represent substantially the same amount of the native Col I, α1 chain and α2 chain.

It is desirable to further purified Col I in the SISH solution to yield a greater than 95% Col I. This purification process involves inducing Col I in the SISH solution to undergo self-assembling to form Col I polymer. The Col I polymer may be used as scaffolds or matrixes in biomedical applications. In addition, the polymer may be isolated or freeze dried and stored for future applications.

The protein assembling process may occur in the SISH solution or on a suitable surface. Such surface may include a wound site or a surface of a biomedical device. Generally, Col I polymer formation may be induced by subjecting the protein solution to a temperature of about 20°C to less than 40°C. However, a temperature of about 34°C to 38°C is particularly suitable. In addition to the temperature treatment, an addition of a salt solution can also accelerate the polymer formation process. A specific example of how the Col I protein polymer is produced in the SISH solution is described in Example 3.

### Example 3

### Col. I polymer formation in SISH solution

A 0.8 mg.ml⁻¹ of SISH prepared according to Example 1 was incubated at a temperature of 37°C, in full humidity, overnight, with or without an addition of various concentrations of (NH₄)₂SO₄. Self-assembly was induced and continued in a slow process (overnight). It was observed that the addition of small amounts of salt increased the rate of assembly at 37°C and could induce fast precipitation even at room temperature (20 to 25°C). However, it was also demonstrated that the addition of larger concentrations of (NH₄)₂SO₄ lead to rapid protein precipitation with loss of the α-helical tertiary structure of the polymeric material with the appearance of a significant fraction of random coils and β-strands as determined by Fourier Transform Infrared Spectroscopy (FTIR), see Table 1. Concomitant with this loss of α-helical motifs there was a loss in tensile strength of the polymer material, as can be seen from the data in table 1 which shows the relationship between tensile strength and failure as a function of secondary structure.

| Treatment | α-Helix | β-sheet | Other | Newtons |
|---|---|---|---|---|
| SISH (no (NH₄)₂SO₄) | 91.2 | 4.7 | 4.1 | 0.434 ± 0.1 |
| SISH + 0.03M (NH₄)₂SO₄ | 79 | 9.5 | 11.5 | 1.341 ± 0.3 |
| SISH + 0.06M (NH₄)₂SO₄ | 78.8 | 10.2 | 11 | 1.017 ± 0.7 |
| SISH + 0.09M (NH₄)₂SO₄ | 79.9 | 8.8 | 11.3 | 0.924 ± 0.2 |
| SISH + 0.16M (NH₄)₂SO₄ | 14.8 | 34.4 | 65.8 | 0.280 ± 0.1 |

In order to determine the surface charge and the stability of the Col I trimer, the Zeta potential was determined for the SISH solution. The Zeta potential is an important and useful indicator of an electronic charge on the surfaces of particles or macroscopic materials in contact with a liquid. The Zeta potential is used to predict and control the stability of colloidal suspensions or emulsions, in the instance case, the protein monomers in the SISH solution. An even charge distribution on the surface of a particle and a similarity in charge across all particles in solution would lead to a stable composition, due to charge repulsion. A change in either condition will cause the particles in solution to interact with one another forming larger assemblies or aggregates culminating in the formation of a two part system, one of which is a precipitate.

For a 0.1 mg.ml⁻¹ solution of SISH in 0.5M acetic acid, pH 2.0, and at 25°C, the Zeta potential was 32.9 mV with a narrow distribution. This value indicated that the solution was homogenous and stable because the charged particles repelled one another and thus overcome the natural tendency to aggregate, even at high concentrations. The addition of 0.06 M (NH₄)₂SO₄ to 0.1 mg/ml solution of SISH caused a significant shift in the value of the Zeta potential to a mean of 1.78 mV and spread between -20 and +30 mV. Such broad distribution indicated one of three things: a heterogeneous population of particles each with a homogeneous but different surface charge, a homogeneous population of particles with a heterogeneous charge distribution or a combination of these two.

Under the same solvent conditions and at 30°C the molecules had two discrete size distributions with 96% particles sizing at 615 nm x 326 nm and 4% at 62 nm × 14.4 nm. Successively increasing the temperature to 35° and 40°C, and caused an increase in size in both populations with a faster increase in the smaller fragment of the population. The larger fibres reached lengths greater than 1 µm. The ratios of length to diameter were maintained close to 4 for the two populations. At 45°C the system reached its denaturation temperature (Tm) and particle sizes returned to a distribution similar to that at 30°C, which is in agreement with the literature. This gradual increase in size in discrete units with temperature and were direct measurements of self assembly and not aggregation.

### Example 4

### Spray Application of the Collagen Matrix

In an alternative embodiment, the present method involves induction of Col I self-assembly directly on a target surface. This can be accomplished by mixing the Col I solution with a salt solution in a mixing device such as the device **10** shown in Fig. 3. The protein solution and the salt solution may be added to reservoir **11** and **13** respectively. A bioactive agent and/or cell containing solution may optionally be added to reservoir **15.** The solutions are mixed in mixing chamber **12** prior to releasing through nozzle **14** on to a target surface. The mixing chamber **12** can be further provide with a port **18** and port extension **20** to place a source of pressurized air in fluid communication with the mixing chamber **12.** Furthermore, a pressure gauge may also be connected to device **10** to control the release of pressurized air into the mixing chamber and regulate the release of the mixture through nozzle **14.** The protein will form a protein polymer covering the surface. If the mixture is sprayed on a wound site, the protein may form a multi-layered scaffold for cell attachment *in situ.*

The Col I-salt-drug mixture can be applied to the surface of the wound or a biomedical device through a variety of different techniques and is dependent on the desired result. One delivery method may be to 'paint' the collagen matrix onto a surface. This would require low pressure (0.034 MPa (5 psi)) to gently coax the collagen matrix out a fanned tip and paint the collagen onto the surface much like an artist would paint on a canvas. Alternatively, the mixture may be sprayed onto a surface at a much higher pressure (0.17 MPa (25 psi)). There could be several different tips that could be used to achieve different types of ejection, i.e. the fanned tip, or spray nozzle tip, or specially designed tips to fit specific body cavities.

### Example 5

### Col Polymer Formation on a Surface

The preparation begins by homogenizing 2 g of SIS in 20 ml of 0.5 M acetic acid at 9500 rpm. The homogenizing is performed three times in 30-second intervals with 60-second breaks where the SISH is chilled on ice. After the homogenizing is complete the final volume is brought up to 40 ml with acetic acid, the vial is capped and shook vigorously. The SISH (collagen type I isolated from SIS) is spun at 4000 rpm for 15 minutes at 4°C. The supernatant contains the acid soluble collagen type I and the pellet is discarded.

The paint method was achieved using a device shown in Fig. 3. A gas pressure gage attached to the spray applicator was adjusted to a pressure of 0.034 MPa (5 psi) or less of gas. A SISH solution containing 0.8 mg.ml⁻¹ of Col I was mixed with various concentrations of salt at a ratio of 10:1. A specially formed fanned tip was attached to the spray applicator. As the gas was expelled the Col I was extruded in a continuous stream on to a bone surface.

For a spray method, the concentration of Col I in the solution (0.8 mg.ml⁻¹) was used. The SISH and salt solution were mixed in a ratio of 10:1 using the dual-syringe device in Fig. 3. A sweeping motion with gentle pushing on the plunger clip and 0.17 MPa (25 psi) of pressure was used for a fine spray, and a lower pressure (0.10 MPa (15 psi)) for a course spray, and a much lower pressure (less than about 0.034 MPa (5 psi)) for an extrusion/flow of the mixture. The bone surface should be at physiologic temperature and semi-dry such that the collagen can stick to the bone, and this may require a drying step with sterile gas.

One spray device suitable for use in the present method is the dual syringe apparatus sold by Micromedics Inc under the trade mark FibriJet. This is a 10:1 mixing ratio device where 10 parts of SISH mixes with 1 part salt and the mix is ejected (sprayed / aerated) via nitrogen at approximately 0.10 MPa (15 psi) using a control unit regulator.

It was determined that a pressurized line of inert gas is optimal for ejection of the Col I mixture. The mechanical force of simply pushing on the syringe plunger was not enough to move the collagen efficiently. Approximately 0.034 MPa (5 psi) provided a sufficient force to push the Col I mixture through the device to achieve a continuous liquid stream. On the other end of the scale as much as 0.17 MPa (25 psi) could be applied to achieve fine spray of the Col I matrix.

Three different inorganic salts delivered via the spray method were tested and ranked based on their ability to form structural collagen on a bony surface. The three are sodium chloride, ammonium sulphate, and sodium phosphate. The best at forming structural collagen was found to be ammonium sulphate followed by sodium phosphate and then sodium chloride. The ranking criteria were as follows: (1) collagen's ability to assemble on a bony surface forming a thick collagen layer on contact and (2) qualitative analysis via Fourier transform infrared spectroscopy (FTIR) to determine α-helical content of the sprayed material and (3) handling during spraying and handling on the bony surface. Shown below in Table 2 is the data complied from FTIR analysis of sprayed SISH under the parameters indicated.

As shown the 30 mM ammonium sulphate shows the cleanest organization of the SISH into a structural α-helical configuration of collagen and also formed the thickest layer on the bone sample. The layer was not too runny and made solid coherence to the bone surface. The 3.0 mM ammonium sulphate also afforded a clean preparation, however a smaller percentage was α-helical. The next preparation was achieved with sodium phosphate, the 2.0 mM solution left a fairly large percentage of the matrix in the α-helical orientation however two separate α-helix peaks were required to get a good fit of the data indicating that perhaps the orientation was not purely α-helix. This preparation was runnier and not as coherent as the ammonium sulphate, resulting in a poorer attachment to the bone sample. The 90 mM sodium phosphate left a poor orientation of α-helix at a mere 45.3%. The next preparation of α-helical collagen was with sodium chloride, this preparation was the sloppiest with most of the collagen simply aggregating with itself and not necessarily adhering to the bone sample. With a 5.2 mM sodium chloride solution there was a significant amount found to be α-helix, however a there was also a lot of other peaks indicating a random architecture. The 52 mM sodium chloride solution proved to be the poorest preparation, with only about 4% being purely α-helix and over 70% of the structure as random turns with no definite organization.

Visual inspection of the collagen forming on the bone samples revealed the limiting step of the collagen in this spray / salt preparation. The collagen could only build so high before it began to reach a limiting factor. As the collagen built on top of the bony surface, there was a point at which no more collagen could be stacked and subsequent collagen simply falls off into surrounding environment. The ammonium sulphate preparation revealed the best stacking of the collagen forming the highest pile and the sodium chloride having the poorest thickness with most of the collagen aggregating and falling into its surroundings.

**Table 2. FTIR data of collagen spray with various salts and concentrations.**

| **Peak no** | **Peak (cm**^{**-1**}**)** | **Percent** | **Peak type** | **Stacking** | **Handling** |
|---|---|---|---|---|---|
| 0.8 mg.ml⁻¹ SISH + 3.0 mM (NH₄)₂SO₄ | | | | | |
| 1 | 1693 | 7.8 | - | Best | Sticky |
| 2 | 1662 | 77.9 | α-helix | | |
| 3 | 1634 | 14.4 | β-sheet | | |

| 0.8 mg.ml⁻¹ SISH + 30 mM (NH₄)₂SO₄ | | | | | |
|---|---|---|---|---|---|
| 1 | 1695 | 5.8 | - | Best | Sticky |
| 2 | 1663 | 80.4 | α-helix | | |
| 3 | 1632 | 13.8 | β-sheet | | |

| 0.8 mg.ml⁻¹ SISH + 2.0 mM Na₃PO₄ | | | | | |
|---|---|---|---|---|---|
| 1 | 1692 | 0.3 | - | Good | Loose |
| 2 | 1661 | 11.0 | α-helix | | |
| 3 | 1656 | 81.5 | α-helix | | |
| 4 | 1631 | 7.2 | β-sheet | | |

| 0.8 mg.ml⁻¹ SISH + 90 mM Na₃PO₄ | | | | | |
|---|---|---|---|---|---|
| 1 | 1685 | 13.0 | β-sheet | Good | Loose |
| 2 | 1661 | 45.3 | α-helix | | |
| 3 | 1634 | 41.2 | β-sheet | | |

| 0.8 mg.ml⁻¹ SISH + 5.2 mM NaCl | | | | | |
|---|---|---|---|---|---|
| 1 | 1695 | 3.8 | - | Poor | Loose |
| 2 | 1662 | 83.4 | α-helix | | |
| 3 | 1642 | 0.7 | Unordered | | |
| 4 | 1631 | 11.7 | β-sheet | | |

| 0.8 mg.ml⁻¹ SISH + 52 mM NaCl | | | | | |
|---|---|---|---|---|---|
| 1 | 1695 | 2.2 | - | Poor | Loose |
| 2 | 1665 | 70.5 | Turns | | |
| 3 | 1661 | 4.2 | α-helix | | |
| 4 | 1649 | 0.5 | α-helix | | |
| 5 | 1634 | 22.6 | β-sheet | | |

There are many potential applications of delivering collagen to a biological surface. The first to consider would be drug delivery. The collagen molecules would act as the workhorse chauffeuring the drug molecules as directed. This could include growth factors or blockers / inhibitors.

Another application would include joint resurfacing. This would be optimal for locations where there are limited bony surfaces to attach a prosthetic device, such as the glenoid cavity of the shoulder. Ideally, both applications could be used in tandem, where a joint could be resurfaced at an accelerated rate through use of a growth factor. The scaffold set up by the sprayed collagen would allow cells to attach and proliferate, biologically resurfacing the joint.

## Claims

1. A method for preparing a biomaterial comprising:
extracting collagen Type I protein from submucosal tissue using an acidic reagent selected from the group consisting of acetic acid, citric acid, or formic acid, in the absence of detergents or enzymes, to produce a solution of collagen fibres wherein greater than 75% of the fibres have an α-helical tertiary structure;
inducing self assembly of the collagen fibres to form the biomaterial.

2. The method of claim 1, wherein the submucosal tissue is intestinal submucosa from a warm-blooded animal.

3. The method of claim 1, wherein the acidic reagent is about 0.1 to about 1.0 M acetic acid.

4. The method of claim 3 wherein the step of inducing self assembly comprises adding a salt solution to the extract.

5. The method of claim 4, wherein the salt solution is selected from the group consisting of ammonium sulphate solution, sodium phosphate solution, phosphate buffered saline (PBS) solution and NaCl solution.

6. The method of claim 5, wherein the salt solution is ammonium sulphate added to a final concentration of about 3.0 mM to about 90 mM.

7. The method of claim 1, wherein the step of inducing self assembly is performed in a presence of a bioactive agent.

8. The method of claim 1 wherein the step of inducing self assembly is performed in a presence of exogenously introduced cells.

9. A protein polymer prepared by the method of claim 6.

10. A method of assembling a collagen type-I matrix on a surface, said method comprising:
applying a composition comprising collagen type I on a surface, wherein said collagen type I is prepared by extracting a naturally occurring extracellular matrix with acetic acid at a pH of about 2-4;
contacting the collagen type I composition with a salt solution to induce formation of a collagen type-I matrix on the surface.

11. A kit for applying a collagen matrix on a surface in situ, said kit comprising
a collagen solution, wherein said collagen is isolated from a natural extracellular matrix using steps comprising extracting the natural matrix with an acidic reagent, selected from the group consisting of acetic acid, citric acid, or formic acid, at a pH of about 2 to about 3.5 and a temperature of less than 20°C; and
a salt solution comprising a salt selected from the group consisting of ammonium sulphate solution, sodium phosphate solution, phosphate buffered saline (PBS) solution and NaCl solution.

12. The kit of claim 11 further comprising a composition comprising a bioactive agent.

13. The kit of claim 12 wherein the bioactive agent is a growth factor.

14. The kit of claim 11 further comprising viable eukaryotic cells.

15. The kit of claim 11 wherein said collagen solution comprises collagen type I isolated from a submucosal matrix by steps comprising homogenizing the submucosal matrix in an acetic acid solution, and isolating the soluble portion.

16. The kit of claim 15 wherein the salt solution is an ammonium sulphate solution.

17. The kit of claim 16 further comprising a device for the mixing and dispensing of the collagen and salt solutions of said kit.

18. The kit of claim 17 wherein the device is provided with a mixing chamber, a nozzle and a plurality reservoirs, wherein each of said reservoirs can be placed in fluid communication with said mixing chamber, said mixing chamber being provided with a nozzle that provides a passageway for material to move from the mixing chamber to the exterior; said mixing chamber further provided with a port and port extension, that when attached to a source of pressurized air, allows fluid communication between the source of pressurized air and the mixing chamber.

19. The kit of claim 18 wherein the collagen solution and the salt solution are prepackaged in the reservoirs of the device.
